# EUROPÄISCHE PATENTSCHRIFT

(11) **EP 0 691 955 B1**
(45) Veröffentlichungstag und Bekanntmachung des Hinweises auf die Patenterteilung: **28.05.1997**
(21) Anmeldenummer: 94913092.6
(22) Anmeldetag: 30.03.1994
(51) Int. Cl.: C07D 211/12, C07D 213/08

(54) **VERFAHREN ZUR HERSTELLUNG VON 3-METHYLPIPERIDIN UND 3-METHYLPYRIDIN DURCH KATALYTISCHE CYCLISIERUNG VON 2-METHYL-1, 5-DIAMINOPENTAN**
PROCESS FOR PREPARING 3-METHYLPIPERIDINE AND 3-METHYLPYRIDINE BY CATALYTIC CYCLISATION OF 2-METHYL-1,5-DIAMINOPENTANE
PROCEDE DE PREPARATION DE 3-METHYLPIPERIDINE ET DE 3-METHYLPYRIDINE PAR CYCLISATION CATALYTIQUE DE 2-METHYL-1,5-DIAMINOPENTANE

(30) Priorität: 02.04.1993 CH 1014/93; 06.01.1994 CH 37/94
(43) Veröffentlichungstag der Anmeldung: 17.01.1996
(73) Patentinhaber: LONZA AG, CH-3945 Gampel/Wallis (CH)
(72) Erfinder: HEVELING, Josef, CH-3904 Naters (CH); ARMBRUSTER, Erich, CH-3904 Naters (CH); SIEGRIST, Walter, CH-3930 Visp (CH)
(74) Vertreter: Weinhold, Peter, Dr.
(86) Internationale Anmeldenummer: EP9401005
(87) Internationale Veröffentlichungsnummer: WO9422824

(56) Entgegenhaltungen:
- EP-A- 0 061 982
- WO-A-90/00546
- WO-A-90/00547
- CH-A- 654 576
- DE-A- 2 519 529
- FR-A- 1 115 492
- GB-A- 2 165 844
- US-A- 3 903 079

## Beschreibung

Die vorliegende Erfindung betrifft ein Verfahren zur Herstellung von 3-Methylpiperidin (MPI) bzw. 3-Methylpyridin (PIC) aus 2-Methyl-1,5-diaminopentan (MPDA).

3-Methylpiperidin findet Verwendung als Vulkanisationsbeschleuniger und als Zusatz für Schmieröl. 3-Methylpyridin wird sowohl als Lösungsmittel als auch als Zwischenprodukt bei der Herstellung von Nicotinsäure eingesetzt.

Aus der PCT-Anmeldung WO 90/00546 ist es bekannt, Mischungen aus 3-Methylpiperidin und 3-Methylpyridin ausgehend von 2-Methyl-1,5-diaminopentan durch Überleiten des gasförmigen Ausgangsproduktes über einen Katalysator aus Metalloxiden bei 500 - 600°C herzustellen. Bevorzugte Katalysatoren sind Kupferchromit, Molybdänoxid oder Vanadiumoxid. Diese Katalysatoren werden vorzugsweise auf einen Träger aufgebracht. Je nach Reaktionstemperatur kann das Mengenverhältnis von Piperidin zu Pyridin mehr auf die eine oder die andere Seite verschoben werden. In dieser Patentschrift wird auch die Möglichkeit erwähnt, saure Oxide wie SiO₂ oder Silicium-Aluminiumoxide ohne weitere Zusätze als Katalysatoren zu verwenden. Die so erzielten Ausbeuten sind aber nur mässig. Über die Kalalysatoraktivitäten über längere Betriebszeiten werden keine Angaben gemacht.

WO-A-9000547, EP-A-0061 982 und DE-A-2 519 529 beschreiben ebenfalls die Herstellung von 3-Methylpyridin aus 2-Methyl-1,5-diaminopentan über 3-Methylpiperidin als Zwischenprodukt. Gemäß WO-A-9000547 verläuft die Reaktion bei einer Temperatur von 400 - 600°C an einem Übergangsmetall-Katalysator, beispielsweise Kupferchromit oder Molybdänoxid.
EP-A-0061 982 verwendet Edelmetall-Katalysatoren wie Palladium, Platin oder Ruthenium auf einem Aluminiumoxid- oder Siliciumdioxid-Träger.
DE-A-2 519 529 verwendet Dehydrierungskatalysatoren wie Nickel, Palladium oder Platin auf einem Trägermaterial. Die Reaktionstemperaturen liegen zwischen 200 und 400°C.

Aus der US-Patentschrift 3 903 079 ist ein Verfahren zur Cycloammonolyse von disubstituierten Alkanen, die primäre Amino- und/oder Hydroxylgruppen aufweisen, bekannt. Als Katalysator findet ein Metallaluminosilikat-Molekularsieb Verwendung. Als Metalle werden bevorzugt Kupfer, Palladium, Mangan, Nickel oder Chrom gewählt. Die Reaktion wird in Gegenwart von Ammoniak durchgeführt. Die erzielten Ausbeuten sind bescheiden. Bei der Herstellung von Piperidin aus 1,5-Pentandiol werden 75% erreicht.

Aufgabe der vorliegenden Erfindung ist es, ein Verfahren zur Herstellung von 3-Methylpiperidin aus 2-Methyl-1,5-diaminopentan zur Verfügung zu stellen, das im kommerziellen Umfang durchgeführt werden kann und hohe Ausbeuten erzielt. Die Katalysatoraktivität soll dabei über lange Zeiten erhalten bleiben. Eine weitere Aufgabe ist die Bereitstellung eines Verfahrens zur Herstellung von 3-Methylpyridin durch weitere Umsetzung des 3-Methylpiperidins über einem Dehydrierungskatalysator.

Erfindungsgemäss wird die Aufgabe durch das Verfahren gemäss Patentanspruch 1 gelöst.

Die erfindungsgemäß bei der Herstellung von 3-Methylpiperidin verwendeten Katalysatoren sind aktiviertes Aluminiumoxid, ein Aluminium-Silicium-Mischoxid oder ein natürlicher oder synthetischer Zeolith.

Wichtig ist, dass sie einen überwiegend sauren Charakter aufweisen und eine spezifische Oberfläche von über 40 m²/g besitzen.
Der saure Charakter ergibt sich aus dem Verhältnis von sauren und basischen Zentren an der Oberfläche, welches erfindungsgemäss über 2 liegen muss. Analytisch werden die sauren Zentren durch irreversible Adsorption von NH₃ bei 80°C, die basischen Zentren durch irreversible Adsorption von CO₂ bei 80°C bestimmt. Als Katalysatoren für das erfindungsgemässe Verfahren werden aktiviertes Al₂O₃, Mischoxide aus Al₂O₃ / SiO₂, oder Zeolithe verwendet. Zeolithe sind kristalline natürliche oder synthetische Aluminiumsilikate, die eine hochgeordnete Struktur mit einem starren dreidimensionalen Netzwerk von SiO₄- und AlO₄-Tetraedern besitzen, welche durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluss von Kationen in den Kristall, z. B. von Alkali- oder Wasserstoffionen, ausgeglichen. Ein Kationenaustausch ist möglich. Die Räume zwischen den Tetraedern sind vor der Dehydration durch Trocknen bzw. Calcinieren von Wassermolekülen besetzt.

Liegt der Zeolith aufgrund der Art seiner Herstellung nicht in der katalytisch aktiven, aciden H-Form vor, sondern z. B. in der Na-Form, dann kann diese durch Ionenaustausch, z. B. mit Ammoniumionen, und anschliessende Calcinierung oder durch Behandlung mit Säuren vollkommen oder partiell in die gewünschte H-Form übergeführt werden.

Die Katalysatoren werden vorzugsweise als Festbettkatalysatoren eingesetzt und das Edukt zweckmässig unter Benützung von Wasserstoff oder einem Inertgas wie Stickstoff als Trägergas durch den Katalysator geleitet.

Die Reaktionstemperatur wird auf 300 - 400°C, vorzugsweise auf 305 - 375°C eingestellt. Der Druck liegt bei 0 - 10 bar, vorzugsweise 0 - 5 bar Überdruck.

Ein Mass für die Belastung von Katalysatoren ist die "Mass Hourly Space Velocity" (MHSV).

Im vorliegenden Fall wird vorteilhaft eine MHSV von 2,1 - 4,2 g Edukt pro g Katalysator und Stunde eingehalten. Das dampfförmige Ausgangsprodukt kann verdünnt werden, vorzugsweise mit N₂ oder H₂.

3-Methylpiperidin kann nach bekannten Dehydrierverfahren in 3-Picolin übergeführt werden. Dabei kann der nach dem Verfahren der Erfindung anfallende 3-Methylpiperidinstrom direkt weiter über einen Dehydrierkatalysator geleitet werden, so dass die Dehydrierung unmittelbar nach der Cyclisierung erfolgt. Dies ist deshalb möglich, weil das 3-Methylpiperidin in einer ungewöhnlich hohen Reinheit anfällt und insbesondere praktisch kein MPDA mehr enthält. Es hat sich nämlich gezeigt, dass die Wirksamkeit der Dehydrierkatalysatoren durch MPDA stark beeinträchtigt wird.

Als Dehydrierungskatalysatoren werden bevorzugt Edelmetalle wie z. B. Pd oder Pt auf einem Träger verwendet. Als besonders vorteilhaft haben sich Dehydrierungskatalysatoren erwiesen, die aus amorphen Silicium-Aluminiumoxiden durch Ionenaustausch mit löslichen Palladiumkomplexen wie [Pd(NH₃)₄]Cl₂ erhältlich sind. Die amorphen Silicium-Aluminiumoxide werden vorteilhaft zunächst entwässert und mit Ammoniak beladen. Der Ionenaustausch mit dem löslichen Palladiumkomplex kann durch Suspendieren des amorphen Oxids in einer Lösung des Komplexes erfolgen. Alternativ kann auch eine Lösung des Komplexes durch eine Packung des amorphen Oxids durchgeleitet werden, wobei aber im Gegensatz zur erstgenannten Methode eine gleichmässige Beladung nur durch vollständigen Austausch erreicht werden kann.

Nach den angegebenen Methoden können auch mit relativ verdünnten Lösungen, beispielsweise 0,01 mol/l [Pd(NH₃)₄]Cl₂, Palladiumgehalte von bis zu 5 Gew.% und höher in einem Schritt erreicht werden.

Die Reaktionstemperatur bei der Dehydrierung liegt vorzugsweise bei 220 - 400°C. Nach einer Ausführungsform wird der Cyclisierungskatalysator direkt auf das Dehydrierungskatalysatorbett aufgebracht und das 2-Methyl-1,5-diaminopentan von oben eingeleitet. Nach einer bevorzugten Ausführungsform werden die Katalysatoren in getrennte Reaktoren eingebracht. Dies erlaubt eine unabhängige Temperaturkontrolle, sowie gegebenenfalls eine unabhängige Katalysatorregeneration.

Die folgenden Beispiele verdeutlichen die Durchführung des erfindungsgemässen Verfahrens. Alle in den Beispielen angegebenen Drucke sind keine Absolutdrucke, sondern Überdrucke bezogen auf Atmosphärendruck.

### Beispiele 1-11

Die in der nachstehenden Tabelle 1 aufgeführten Beispiele für die Cyclisierung von Methyldiaminopentan (MPDA) zu Methylpiperidin (MPI) wurden wie folgt durchgeführt. Die Beispiele 1, 2 und 3 sind Vergleichsbeispiele (nicht erfindungsgemäss).

In einen Reaktor (13 mm ⌀) wurden 3 g Katalysator (Korngrösse: 0,32 - 1 mm) eingefüllt. MPDA wurde verdampft und mit einem Trägergasstrom von 15 ml/min N₂ bei einem Druck von 5 bar über den Katalysator geleitet. Das Katalysatorbett wurde schrittweise aufgeheizt und die Reaktion gaschromatographisch verfolgt. Je aktiver der Katalysator, desto geringer ist die für die Cyclisierung von MPDA zu MPI erforderliche Temperatur. Über die für eine möglichst hohe MPI-Ausbeute erforderlichen Temperaturen und unter Berücksichtigung der Katalysatorbelastung (MHSV) lässt sich die Aktivität der verwendeten Katalysatoren untereinander vergleichen.

Die Tabelle wird ergänzt durch Charakterisierungsdaten zu den verwendeten Katalysatoren.

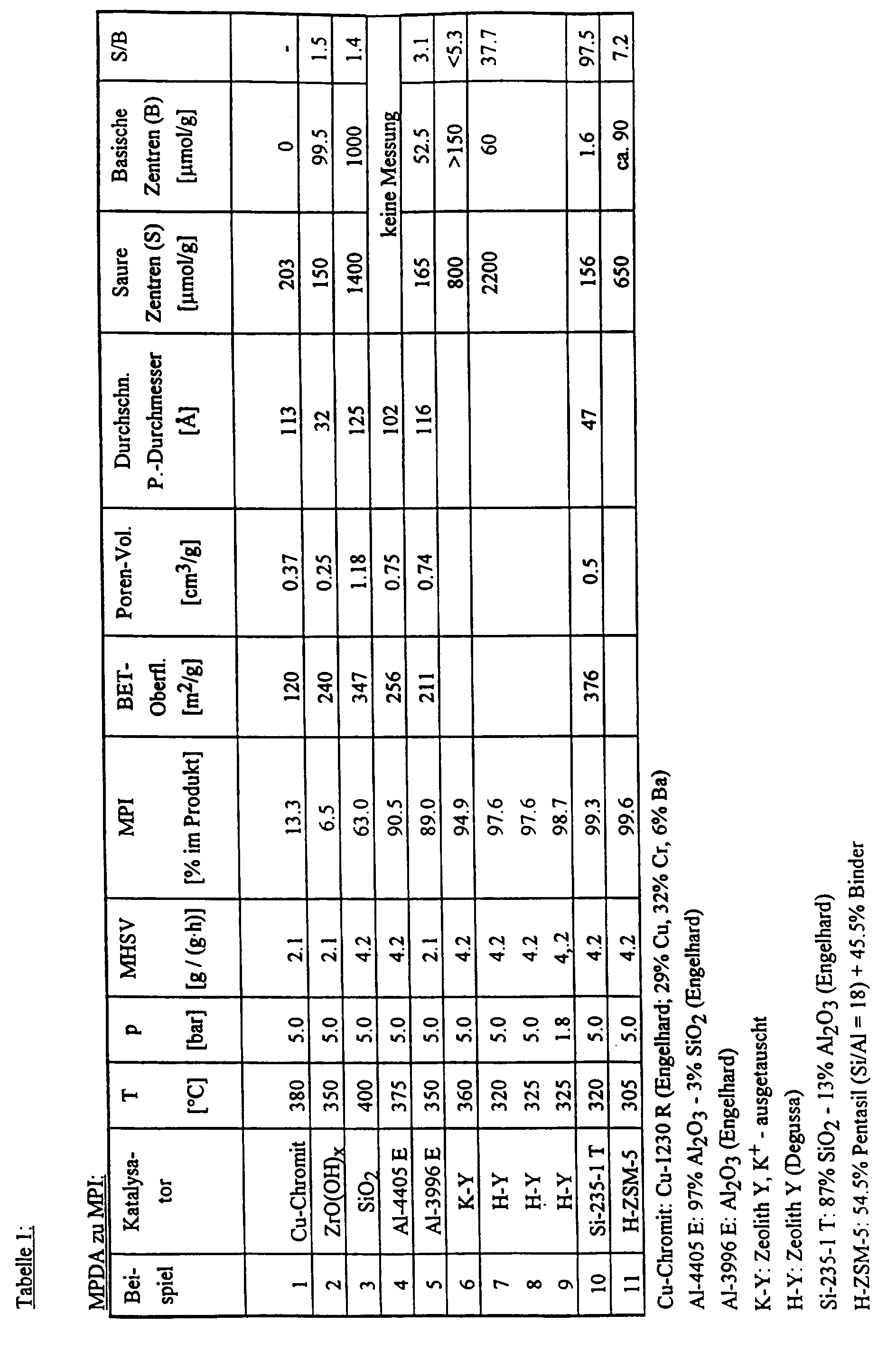

### Beispiel 12:

MPDA zu 3-Picolin:
In einen Reaktor (13 mm ⌀) wurden 4 g eines Pd-Katalysators (1% Pd/Al₂O₃) eingefüllt und darüber 3 g H-ZSM-5. (Das Edukt wurde immer von oben in den Reaktor eingespeist.) Die Reaktionsbedingungen waren: Temperatur: 305 - 320°C, 15 ml/min N₂, Druck: 5 bar. Im Temperaturbereich 305 - 320°C und bei einer MHSV von 0.6 g/(g·h) wurden Ausbeuten von bis zu 97% 3-Picolin erreicht, wobei als einziges weiteres Produkt 2.9% MPI gefunden wurden. Es erfolgte also ein vollständiger Umsatz des MPDA zu erwünschten Produkten. Innerhalb von 10 Tagen wurde keine Desaktivierung der Katalysatoren beobachtet. Statt N₂ kann auch H₂ als Trägergas verwendet werden.
Die neue Fahrweise führt also zu einer wesentlichen Verbesserung der Aktivität, Selektivität und der Katalysatorstandzeit.

### Beispiel 13

Herstellung von 3-Picolin mit zwei getrennten Reaktoren und kommerziellem MPDA (MPDA zu 3 Picolin in 2 Stufen mit Isolierung von MPI):
**1. Stufe:** In einen Reaktor (13 mm ⌀) wurden 3 g ZSM-5 in der Ammonium-Form (Korngrösse: 0.5 - 1 mm) eingefüllt. MPDA wurde verdampft und mit einem Trägergasstrom von 15 ml/min N₂ bei einem Druck von 5 bar und einer Temperatur von 335°C über den Katalysator geleitet. Die MHSV betrug 4.2 g MPDA pro Gramm Katalysator pro Stunde. Das verwendete MPDA war ein kommerzielles Produkt, das von der Firma Du Pont de Nemours unter dem Handelsnamen Dytek A erhältlich ist. Der Versuch lief über 280 Stunden. Eine Desaktivierung des Katalysators war nicht zu beobachten. Das Produkt wurde kondensiert, und das gebildete Ammoniak konnte entweichen. Die Ausbeuten an MPI waren praktisch quantitativ (>99.5%).
**2. Stufe:** In einen Reaktor (13 mm ⌀) wurden 10 g eines Pd-MgCl₂/Al₂O₃-Dehydrierkatalysators eingefüllt. Das MPI aus dem Versuch vorher wurde dampfförmig mit einem Trägergasstrom von 15 ml/min N₂ bei einem Druck von 1 bar und einer Temperatur von 280°C über den Katalysator geleitet. Die MHSV betrug 0.23 g MPI pro Gramm Katalysator pro Stunde. Der Versuch lief über 190 Stunden. Eine Desaktivierung des Katalysators war nicht zu beobachten. Nach 190 h wurde gaschromatographisch folgende Produktzusammensetzung festgestellt: 99.3% 3-Picolin, 0.4% MPI.

### Beispiel 14:

Herstellung von 3-Picolin mit zwei getrennten Reaktoren und kommerziellem MPDA (MPDA zu 3-Picolin in 2 Stufen ohne Isolierung von MPI):
In einen Reaktor (13 mm ⌀) wurden 3 g NH₄-ZSM-5 (Korngrösse: 0.5 - 1 mm) eingefüllt. MPDA wurde verdampft und mit einem Trägergasstrom von 15 ml/min N₂ bei einem Druck von ca. 1 bar und einer Temperatur von 320°C über den Katalysator geleitet. Die MHSV lag zwischen 1 und 2 g MPDA pro Gramm ZSM-5 pro Stunde. Das verwendete MPDA war ein kommerzielles Produkt, das von der Firma Du Pont de Nemours unter dem Handelsnamen Dytek A erhältlich ist. Das Produkt aus dem Cyclisierungs-Reaktor wurde in der Gasphase gehalten und direkt auf einen zweiten Reaktor geleitet. Dieser Reaktor enthielt 12 g eines Dehydrierkatalysators der Zusammensetzung Pd + MgCl₂ auf einem Al₂O₃-Träger (Korngrösse: 0.32 - 1 mm). Die Reaktionsbedingungen waren 280°C und ca. 1 bar. Das Kondensat aus dem Dehydrierreaktor enthielt nach 220 Stunden Reaktionszeit 99.1% 3-Picolin und 0.9% MPI (gaschromatographisch). Eine Desaktivierung der beiden Katalysatoren über die Reaktionszeit war nicht zu beobachten.

### Beispiel 15 (Vergleichsbeispiel):

Herstellung eines 1%Pd/Al₂O₃-Katalysators durch Imprägnieren:
Zu 540 g deionisiertem Wasser wurden 6.3 g Pd(NO₃)₂-Hydrat (Heraeus) und 15.3 g konz. HCI gegeben. Es stellte sich ein pH von 0.7 ein. Diese Lösung wurde zu 250 g Al₂O₃ (Al-4191 E 1/16" von Engelhard) gegeben, das zuvor mit deionisiertem Wasser angefeuchtet worden war. Die Imprägnierzeit betrug 3 Tage. Anschliessend wurde die Lösung abdekantiert und der Katalystor 20 h bei 150°C getrocknet. Dann wurde für 2 h bei 550°C im Umluftofen kalziniert. Der Katalysator wurde granuliert und die Siebfraktion 0.315 - 1 mm gesammelt.

### Beispiel 16 (Vergleichsbeispiel):

Herstellung eines 3%Pd/Al₂O₃-Katalysators durch Imprägnieren:
Al₂O₃ (Al-3996 R von Engelhard) wurde granuliert und die Siebfraktion 0.315 - 1 mm verwendet. Es wurden drei Imprägnierlösungen aus 150 g deionisiertem Wasser, 1.8 g Pd(NO₃)₂-Hydrat (Heraeus) und 2.36 g konz. HCI hergestellt. Es stellte sich ein pH von *ca*. 0.8 ein. 70 g des Trägers wurden nacheinander je 24 h mit diesen drei Imprägnierlösungen imprägniert, wobei der Katalysator nach jedem Imprägnierschritt mit 100 ml deionisiertem Wasser gewaschen, 2 h bei 150°C im Vakuumofen getrocknet und 2 h bei 550°C im Umluftofen kalziniert wurde.

### Beispiel 17 (Vergleichsbeispiel) :

Herstellung eines 4%Pd/Al₂O₃-Katalysators durch Imprägnieren:
Es wurden zwei Imprägnierlösungen aus 150 g deionisiertem Wasser, 1.25 g Pd(NO₃)₂-Hydrat (Heraeus) und 2.24 g konz. HCl hergestellt. Es stellte sich ein pH von 0.8 ein. 50 g des Katalysators aus Beispiel 2 wurden nacheinander mit diesen Imprägnierlösungen imprägniert, wobei der Katalysator nach jedem Schritt mit 100 ml deionisiertem Wasser gewaschen, 2 h bei 150°C im Vakuumofen getrocknet und 2 h bei 550°C im Umluftofen kalziniert wurde.

### Beispiel 18:

Herstellung eines 5%Pd-SiO₂/Al₂O₃-Katalysators durch Ionenaustausch mit [Pd(NH₃)₄]²⁺:
Der Si/Al-Oxid-Träger (13 Gew.-% Al₂O₃) Si-235-1 T von Engelhard wurde granuliert (0.315 - 1 mn). 50 g des Granulats wurden in einem Quarzrohr bei 400°C im N₂-Strom 12 h entwässert. Über die abgekühlte Probe wurde für 1 h trockenes Ammoniakgas (36 g) geleitet. Es wurde eine 0.01 molare [Pd(NH₃)₄]Cl₂-Lösung hergestellt: Auf 100 ml 0.84 molare wässrige NH₃-Lösung wurden 0.375 g PdCl₂ gegeben und für 15 min bei 85°C gerührt. Nach den Abkühlen wurde die gewünschte Molarität durch Wasserzugabe eingestellt. 20 g des vorbehandelten Trägers wurden 24 h mit 2542 ml der 0.01 molaren Pd-Salz-Lösung gerührt. Anschliessend wurde der Katalysator 6 mal mit je 500 ml deionisiertem Wasser gewaschen und 24 h bei 120°C getrocknet. Der Katalysator enthielt *ca.* 5 Gew.-% Pd.

### Beispiel 19:

Herstellung eines 5%Pd-SiO₂/Al₂O₃-Katalysators durch Ionenaustausch mit [Pd(NH₃)₄]²⁺:
150 g des Si/Al-Oxid-Trägers (15 Gew.-% Al₂O₃) Si-HP-87-069 T von Engelhard wurden in einem Quarzrohr bei 400°C im N₂-Strom 12 h entwässert. Über die abgekühlte Probe wurde für 1 h trockenes Ammoniakgas (60 g) geleitet.
70 g des vorbehandelten Trägers wurde 20 h mit 3720 ml einer 0.01 molaren Pd-Salz-Lösung (hergestellt nach Beispiel 18) gerührt. Anschliessend wurde der Katalysator 6 mal mit 1000 ml deionisiertem Wasser gewaschen und 15 h bei 120°C getrocknet. Der Katalysator enthielt *ca.* 5 Gew-%Pd.

### Beispiel 20:

Herstellung eines 3%Pd-SiO₂/Al₂O₃-Katalysators durch Ionenaustausch mit [Pd(NH₃)₄]²⁺:
120 g des Si/Al-Oxid-Trägers (15 Gew.-% Al₂O₃) Si-HP-87-069 T von Engelhard wurden in einem Quarzrohr bei 400°C im N₂-Strom 12 h entwässert. Über die abgekühlte Probe wurde für 1 h trockenes Ammoniakgas (35 g) geleitet.
35 g des vorbehandelten Trägers wurden 24 h mit 1030 ml einer 0.01 molaren Pd-Salz-Lösung (hergestellt nach Beispiel 18) gerührt. Anschliessend wurde der Katalysator 6 mal mit je 1000 ml deionisiertem Wasser gewaschen und 24 h bei 120°C getrocknet. Der Katalysator enthielt *ca.* 3 Gew.-% Pd.

### Beispiel 21:

Herstellung eines 1%Pd-SiO₂/Al₂O₃-Katalysators durch Ionenaustausch mit [Pd(HH₃)₄]²⁺:
76.5 g des Si/Al-Oxid-Trägers (15 Gew.-% Al₂O₃) Si-HP-87-069 T von Engelhard wurden in einem Quarzrohr bei 400°C im N₂-Strom 12 h entwässert. Über die abgekühlte Probe wurde für 1 h trockenes Ammoniakgas (69 g) geleitet.
Es wurde eine 0.0033 molare [Pd(NH₃)₄]Cl₂-Lösung hergestellt: Auf 100 ml 0.84 molare wässrige NH₃-Lösung wurden 0.375 g PdCl₂ gegeben und für 15 min bei 85°C gerührt. Nach den Abkühlen wurde die gewünschte Molarität durch Wasserzugabe eingestellt.
35 g des vorbehandelten Trägers wurden 24 h mit 1030 ml der 0.0033 molaren Pd-Salz-Lösung gerührt. Anschliessend wurde der Katalysator 6 mal mit je 1000 ml deionisiertem Wasser gewaschen und 24 h bei 120°C getrocknet. Der Katalysator enthielt *ca.* 1 Gew.-% Pd.

### Beispiel 22:

Herstellung eines 1%Pd-SiO₂/Al₂O₃-Katalysators durch Behandlung mit PdCl₂:
150 g des Si/Al-Oxid-Trägers (15 Gew.-% Al₂O₃) Si-HP-87-069 T von Engelhard wurden in einem Quarzrohr bei 400°C im N₂-Strom 12 h entwässert. Über die abgekühlte Probe wurde für 1 h trockenes Ammoniakgas (60 g) geleitet.
Analog zu Beispiel 18 wurde eine 0.015 molare PdCl₂-Lösung hergestellt.
35 g des vorbehandelten Trägers wurden 24 h mit 1000 ml der 0.015 molaren PdCl₂-Lösung gerührt. Anschliessend wurde der Katalysator 2 mal mit 500 ml deionisiertem Wasser gewaschen und 24 h bei 120°C getrocknet. Der Katalysator enthielt *ca.* 1.4 Gew.-% Pd; der Chlorgehalt lag unter 0.01%.

### Beispiel 23:

Herstellung eines 6%Pd-SiO₂/Al₂O₃-Katalysators durch Ionenaustausch mit [Pd(NH₃)₄]²⁺ in einer Glaskolonne:
900 g des Si/Al-Oxid-Trägers (15 Gew.-% Al₂O₃) Si-HP-87-069 T 1/8" von Engelhard wurden in einem Quarzrohr bei 400°C im N₂-Strom 12 h entwässert. Über die abgekühlte Probe wurde für 1.25 h trockenes Ammoniakgas (155 g) geleitet.
Es wurden 67.6 l einer 0.01 molaren [Pd(NH₃)₄]Cl₂-Lösung hergestellt: Auf 31.71 0.84 molare wässrige NH₃-Lösung wurden 119 g PdCl₂ gegeben und bei 85°C gerührt bis die Lösung klar ist. Nach den Abkühlen wurde die gewünschte Molarität durch Zugabe von weiteren 35.9 l Wasser eingestellt.
Der vorbehandelten Träger wurde in eine Glaskolonne gefüllt (Länge: 115 cm, Durchmesser: 6.5 cm), und während 15 h wurde die Pd-Lösung mittels einer Schlauchpumpe im Kreislauf über den Träger gepumpt (60 l/h). Anschliessend wurde der Katalysator 6 mal mit je 9 l deionisiertem Wasser in einem Rührgefäss gewaschen und 24 h bei 120°C im Umluftofen getrocknet. Der gelbe Katalysator (982 g) enthielt *ca.* 6 Gew.-% Pd.

### Beispiel 24:

Herstellung eines 6%Pd-SiO₂/Al₂O₃-Katalysators durch Ionenaustausch eines nach dem Sol-Gel-Verfahren hergestellten Si/Al-Oxids mit [Pd(NH₃)₄]²⁺:
Der Si/Al-Oxid-Pulver (13 Gew.-% Al₂O₃) MS 13/110 von der Firma Grace wurde zu Tabletten (9 mm ⌀) gepresst. Die Tabletten wurden gebrochen und die Siebfraktion von 0.315 - 1 mm gesammelt. 95 g des Granulats wurden in einem Quarzrohr bei 400°C im N₂-Strom (250 ml/min) 12 h entwässert. Über die abgekühlte Probe wurde für 1 h trockenes Ammoniakgas (58 g) geleitet.
80 g des vorbehandelten Trägers wurde 24 h mit 10.1 l einer 0.01 molaren Pd-Salz-Lösung (hergestellt nach Beispiel 18) gerührt. Anschliessend wurde der Katalysator 6 mal mit 1000 ml deionisiertem Wasser gewaschen und 24 h bei 120°C getrocknet. Der Katalysator enthielt *ca.* 6 Gew-%Pd.

### Beispiel 25:

Herstellung eines 2%Pd-ZSM-5 - Katalysators durch Ionenaustausch mit [Pd(NH₃)₄]²⁺:
Ein Pentasil-Zeolith (3.1 Gew.-% Al₂O₃) in der Korngrösse 0.315 - 1 mm enthielt 30% Aluminiumoxid als Binder. 60 g des Produktes wurden in einem Quarzrohr bei 400°C im N₂-Strom 12 h entwässert. Über die abgekühlte Probe wurde für 1 h trockenes Ammoniakgas (35 g) geleitet.
20 g des vorbehandelten Pentasils wurde mit 420 ml einer 0.01 molaren Pd-Salz-Lösung (hergestellt nach Beispiel 18) ausgetauscht. Anschliessend wurde der Zeolith 6 mal mit 250 ml deionisiertem Wasser gewaschen und 24 h bei 120°C getrocknet. Der Katalysator enthielt *ca.* 2 Gew.-% Pd.

### Beispiele 26 - 33 (Tabelle 2)

Dehydrierung von 3-Methylpiperidin (MPI) zu 3-Picolin (PIC):
In einen Reaktor (13 mm ⌀) wurden 3 - 10 g Katalysator (Korngrösse: 0.315 - 1 mm) eingefüllt. MPI wurde verdampft und bei der in Tabelle 2 angegebenen Reaktortemperatur über den Katalysator geleitet (p ≈ 1 bar). In den meisten Fällen wurde zusätzlich ein Wasserstoffstrom von 15 ml/min eingestellt. Der Produktstrom wurde gaschromatographisch analysiert. Die in der Tabelle 2 angegebenen Analysenwerte wurden erhalten, nachdem sich konstante Reaktionsbedingungen eingestellt hatten (>20 h).

**Tabelle 2:**

| **Beispiel** | **Katalysator** | | **Zusatz** | **T [°C]** | **MHSV [1/h]** | **PIC** | **MPI** |
|---|---|---|---|---|---|---|---|
| | **Typ** | **Herstellung** | | | | **[GC-Fl.-%]** | |
| 26 | 1%Pd-M_{g}Cl₂/Al₂O₃ | DOS 3410542 | - | 270 | 0.25 | 93.6 | 4.3 |
| 27 | 1%Pd/Al₂O₃ | Beispiel 15 | 15 ml/min H₂ | 280 | 0.44 | 97.0 | 2.4 |
| 28 | 4%Pd/Al₂O₃ | Beispiel 17 | 15 ml/min H₂ | 270 | 0.44 | 98.8 | 1.2 |
| 29 | 5%Pd-SiO₂/Al₂O₃ | Beipiel 18 | 15 ml/min H₂ | 280 | 1.76 | 99.3 | - |
| 30 | 3%Pd-SiO₂/Al₂O₃ | Beispiel 20 | 15 ml/min H₂ | 280 | 1.76 | 99.2 | 0.3 |
| 31 | 1%Pd-SiO₂/Al₂O₃ | Beispiel 21 | 15 ml/min H₂ | 280 | 1.76 | 98.4 | 0.2 |
| -"- | -"- | -"- | -"- | -"- | 0.88 | 99.0 | 0.2 |
| -"- | -"- | "-" | -"- | 290 | 0.44 | 99.5 | 0.2 |
| 32 | 1.4%Pd-SiO₂/Al₂O₃ | Beispiel 22 | 15 ml/min H₂ | 280 | 1.76 | 57.8 | 40.6 |
| 33 | 6%Pd-SiO₂/Al₂O₃ | Beispiel 24 | 15 ml/min H₂ | 280 | 1.76 | 99.3 | 0.3 |
| -"- | -"- | -"- | -"- | -"- | -"- | 98.4 | 1.2 |

Es fällt auf, dass der nach einer früheren Patentschrift (DOS 3410542) erhaltene imprägnierte Pd-Mg-Katalysator (Beispiel 26) sowie die durch Imprägnieren von Aluminiumoxid mit Pd erhaltenen Katalysatoren (Beispiele 27 u. 28) weniger 3-Picolin und mehr nicht-umgesetztes MPI im Produktstrom liefern als die Katalysatoren aus den Beispielen 15 - 17 und 19. Dies ist umso überraschender, als die Versuche mit den imprägnierten Katalysatoren bei einer geringen Katalysatorbelastung gefahren wurden. Die Katalysatoren der Beispiele 29 - 31 und 33 wurden durch Ionenaustausch von Silicium-Aluminium-Oxid mit [Pd(NH₃)₄]Cl₂ erhalten. Die Aktivität kann zu einem gewissen Mass über den Austauschgrad kontrolliert werden (vergl. Beispiele 29 - 31 mit 5%, 3% und 1% Palladium im ausgetauschten Katalysator). In Beispiel 32 wurde ein Katalysator eingesetzt, bei dem der Träger nicht mit [Pd(NH₃)₄]Cl₂, sondern mit PdCl₂ behandelt worden war. Dieser Katalysator zeigte eine viel geringere Aktivität als die mit [Pd(NH₃)₄]Cl₂ behandelten.

### Beispiele 34 - 40:

In einen Reaktor (13 mm ⌀) wurden 3 - 10 g Katalysator (Korngrösse: 0.315 - 1 mm) eingefüllt. Als Edukt wurde eine Rohware ("MPI Roh") verwendet, die aus einem Gemisch folgender Zusammensetzung hergestellt worden war: 74.9% MPI, 13.9% 2-Methyl-1,5-diaminopentan (MPDA), 5.1% organische Verunreinigungen (hauptsächlich Methylcyclopentandiamine) und 6.1% Wasser. Der Herstellung der Rohware erfolgte durch katalytische Cyclisierung des im Ausgangsgemisch enthaltenen MPDA gemäss Beispielen 1-11. Nach der Cyclisierung hatte das "MPI Roh" folgende Zusammensetzung: 89.9% MPI, 4.0% organische Verunreinigungen und 6.1% Wasser. Dieses Ausgangsmaterial wurde verdampft und bei den in Tabelle 3 angegebenen Reaktortemperaturen über die in derTabelle angegebenen Katalysatoren geleitet (p ≈ 1 bar). In den meisten Fällen wurde zusätzlich ein Wasserstoffstrom von 15 ml/min eingestellt. Der Produktstrom wurde gaschromatographisch analysiert.

**Tabelle 3:**

| **Beispiel** | **Katalysator** | | **Zusatz** | **T [°C]** | **MHSV [1/h]** | **PIC** | **MPI** |
|---|---|---|---|---|---|---|---|
| | **Träger** | **Herstellung** | | | | **[GC-Fl.-%]** | |
| 34 | 1%Pd-M_{g}Cl₂/Al₂O₃ | DOS 3410542 | 15 ml/min H₂ | 280 | 0.44 | 96.0 | 0.2 |
| -"- | -"- | -"- | -"- | -"- | 1.76 | 84.5 | 10.1 |
| 35 | 5%Pd-SiO₂/Al₂O₃ | Beispiel 18 | 60 ml/min NH3 | 280 | 1.76 | 95.5 | 0.3 |
| 36 | 5%Pd-SiO₂/Al₂O₃ | -"- | - | 285 | 1.76 | 97.9 | - |
| -"- | -"- | -"- | - | -"- | 3.52 | 93.4 | 2.2 |
| 37 | -"- | -"- | 15 ml/min H₂ | 280 | 3.52 | 93.9 | 1.5 |
| 38 | 5%Pd-SiO₂/Al₂O₃ | Beispiel 19 | 15 ml/min H₂ | 280 | 3.52 | 96.0 | 0.4 |
| 39 | 3%Pd-SiO₂/Al₂O₃ | Beispiel 20 | 15 ml/min H₂ | 280 | 1.76 | 96.2 | 0.2 |
| -"- | -"- | -"- | -"- | 290 | -"- | 96.5 | 0.3 |
| 40 | 6%Pd-SiO₂/Al₂O₃ | Beispiel 24 | 15 ml/min H₂ | 280 | 3.52 | 95.2 | 0.4 |

Es fällt auf, dass der nach einer früheren Patentschrift (DOS 3410542) erhaltene imprägnierte Pd-Mg-Katalysator (Beispiel 34) bei einer MHSV von 1.76 weniger 3-Picolin und mehr nicht-umgesetztes MPI im Produktstrom enthält als die Katalysatoren aus den Beispielen 35 - 40. Die Katalysatoren der Beispiele 35 - 40 wurden durch Ionenaustausch von Silicium-Aluminium-Oxid mit [Pd(NH₃)₄]Cl₂ erhalten. Diese Katalysatoren weisen eine erheblich höhere Aktivität auf und selbst bei einer MHSV von 3.52 lassen sich noch MPI-Umsätze von über 99.5% erzielen.
Der Katalysator von Beispiel 40 wurde durch Ionenaustausch eines Silicium-Aluminium-Oxids erhalten, dass nach dem Sol-Gel-Verfahren hergestellt worden war.
In Beispiel 35 wurde Ammoniak zudosiert. Der Versuch zeigt, dass der bei der Cyclisierung von MPDA zu MPI freigesetzte Ammoniak die Reaktion nicht stört. Die Reaktion läuft auch ab, wenn kein Wassserstoff-Trägergas zudosiert wird (Beispiel 36).

### Beispiel 41:

Pd-ausgetauschter Zeolith als Katalysator:
In einen Reaktor (13 mm ⌀) wurden 10 g des Pd-ZSM-5 - Katalysators aus Beispiel 25 eingefüllt (Korngrösse: 0.315 - 1 mm). MPI wurde verdampft und bei einer Reaktortemperatur von 280°C und einer MHSV von 0.44 über den Katalysator geleitet (p ≈ 1 bar). Der Produktstrom wurde gaschromatographisch analysiert (GC-Fl.-%). Nach einer Reaktionszeit von 21 h enthielt der Produktstrom 99.2% PIC und 0.8% unumgesetztes MPI. Nach einer Reaktionszeit von 213 h enthielt der Produktstrom 93.15% PIC und 6.85% unumgesetztes MPI.

### Beispiel 42:

Bei diesem Versuch wurde eine isotherme Reaktionsführung angestrebt.
Hierzu wurden in einen Reaktor (21 mm ⌀) 27 g des Katalysators aus Beispiel 19 eingefüllt (Korngrösse: 0.315 - 1 mm). Der Katalysator wurde mit 53 g des Katalysatorträgers so verdünnt, dass der Katalysator an der Reaktoreingangsseite am stärksten verdünnt war, an der Ausgangsseite unverdünnt vorlag und das Konzentrationsgefälle entlang dem Katalysatorbett ungefähr einer Exponentialfunktion folgte. Das Edukt hatte folgende Zusammensetzung: 92.7% MPI, 6.5% Wasser, 0.8% organische Verunreinigungen. Das Edukt wurde verdampft und mit einer MHSV von 4.73 bezogen auf den aktiven Katalysator (entsprechend 1 g Edukt pro ml Kat.-Bett pro h) über das Katalysatorbett geleitet (p = 0.11 bar). Der Produktstrom wurde gaschromatographisch analysiert (GC-Fl:%). Der Umsatz war quantitativ, und nach 339 h enthielt der organische Anteil des Produktes noch 99.3% PIC und 0.7% organische Verunreinigungen. Aufgrund der Endothermie der Reaktion stellte sich im Zentum des Reaktors eine Temperatur von ca. 240°C ein (Wandtemperatur 280-300°C). Am Ende des Katalysatorbetts betrug die Temperatur 300°C über den gesamten Querschnitt des Reaktors. Nach einer Reaktionszeit von 362 h wurde als Edukt reines, wasserfreies MPI eingesetzt. Nach 454 h enthielt der Produktstrom 99.2% PIC, 0.4% unumgesetztes MPI und 0.4% organische Verunreinigungen.

### Beispiel 43:

2-Methyl-1,5-diaminopentan (MPDA) zu 3-Picolin kontinierlich in 2 Stufen:
In einen Reaktor (13 mm ⌀) wurden 3 g SiO₂/Al₂O₃-Granulat (Si-HP-87-069 T von Engelhard) in der Korngrösse 0.315 - 1 mm eingefüllt. MPDA wurde verdampft und mit einem Trägergasstrom von 15 ml/min H₂ bei einem Druck von *ca*. 1 bar und einer Reaktortemperatur von 320°C über den Katalysator geleitet und zu MPI cyclisiert. Das verwendete MPDA war ein kommerzielles Produkt, das von der Firma Du Pont de Nemours unter dem Handelsnamen Dytek A erhältlich ist. Das Produkt aus dem Cyclisierungs-Reaktor wurde in der Gasphase gehalten und direkt auf einen zweiten Reaktor geleitet. Dieser Reaktor enthielt 3 g des Dehydrierkatalysators aus Beispiel 18 (Korngrösse: 0.32-1 mm). Die Reaktortemperator war 280°C, der Druck 1 bar. Im Laufe des Versuchs wurde das Edukt von MPDA zu MPI und dann zu einer Rohware (3-MP Roh) gewechselt, die aus einem Gemisch folgender Zusammensetzung besteht: 74.9% MPI, 13.9% MPDA, 5.1% organische Verunreinigungen (hauptsächlich Methylcyclopentandiamine) und 6.1% Wasser. Die Ergebnisse mit den zugehörigen MHSV's (MHSV bezogen auf Reaktor 1) sind in der folgenden Tabelle 4 zusammengestellt:

**Tabelle 4:**

| **Edukt** | **MHSV [1/h]** | **PIC** | **MPI** | **Laufzeit [h]** | **Desaktivierung [PIC %/h]** |
|---|---|---|---|---|---|
| | | **[GC-Fl.-%]** | | | |
| Dytek A | 2.1 | 99.7 | - | 71 | 0 |
| -"- | 3.15 | 99.6 | 0.2 | 25 | 0 |
| -"- | 4.2 | 98.6 | 1.4 | 48 | 0 |
| MPI | 4.1 | 95.2 | 3.8 | 3 | - |
| -"- | 3.52 | 98.6 | 0.6 | 92 | 0 |
| 3-MP Roch | 4.2 | 93.9 | 1.5 | 70 | 0.0172 |

### Beispiel 44:

2-Methyl-1,5-diaminopentan (MPDA) zu 3-Picolin, kontinierlich in 2 Stufen:
In einen Reaktor (13 mm ⌀) wurden 3 g SiO₂/Al₂O₃-Granulat (Si-HP-87-069 T von Engelhard) in der Korngrösse 0.315 - mm eingefüllt. MPDA wurde verdampft und mit einem Trägergasstrom von 15 ml/min H₂ bei einem Druck von *ca.* 1 bar und einer Reaktortemperatur von 320°C über den Katalysator geleitet und zu MPI cyclisiert. Das verwendete MPDA war ein kommerzielles Produkt, das von der Firma Du Pont de Nemours unter dem Handelsnamen Dytek A erhältlich ist. Das Produkt aus dem Cyclisierungs-Reaktor wurde in der Gasphase gehalten und direkt auf einen zweiten Reaktor geleitet. Dieser Reaktor enthielt 3 g des Dehydrierkatalysators aus Beispiel 20 (Korngrösse: 0.315 - 1 mm). Die Reaktortemperator war 280°C, Druck 1 bar. Im Laufe des Versuchs wurde das Edukt von MPDA zu einer Rohware (3-MP Roh) gewechselt, die aus einem Gemisch folgender Zusammensetzung besteht: 74.9% MPI, 13.9% MPDA, 5.1% organische Verunreinigungen (hauptsächlich Methylcyclopentandiamine) und 6.1% Wasser. Die Ergebnisse mit den zugehörigen MHSV's (MHSV bezogen aufReaktor 1) sind in der folgenden Tabelle 5 zusammengestellt:

**Tabelle 5:**

| **Edukt** | **MHSV [1/h]** | **PIC** | **MPI** | **Laufzeit [h]** | **Desaktivierung [PIC %/h]** |
|---|---|---|---|---|---|
| | | **[GC-Fl.-%]** | | | |
| Dytek A | 2.1 | 97.5 | 1.4 | 117 | 0.0204 |
| -"- | 1.0 | 98.2 | 0.7 | 18 | 0 |
| 3-MP Roh | -"- | 97.6 | 0.2 | 119 | 0.0248 |

### Beispiel 45:

3-MP Roh zu 3-Picolin, kontinierlich in 2 Stufen mit zwischengeschaltenem Teerabscheider:
Im Unterschied zu Beispiel 44 hatte das Edukt eine andere Zusammensetzung und zwischen 1. und 2. Reaktor wurde ein Teerabscheider eingebaut.
In einen Reaktor (13 mm ⌀) wurden 3 g SiO₂/Al₂O₃-Granulat (Si-HP-87-069 T von Engelhard) in der Korngrösse 0.315 - 1 mm eingefüllt.
Das Edukt war eine Rohware (3-MP Roh) folgender Zusammensetzung: 45.8% MPI, 29.9% MPDA, 9.8% organische Verunreinigungen (hauptsächlich Methylcyclopentandiamine) und 14.5% Wasser. Das Edukt wurde verdampft und mit einem Trägergasstrom von 15 ml/min H₂ und einer MHSV von 4.2 bei einem Druck von *ca.* 1 bar und einer Reaktortemperatur von 320°C über den Reaktor geleitet. Das Produkt aus dem Cyclisierungs-Reaktor wurde über einen Teerabscheider (115°C) geleitet und direkt auf einen zweiten Reaktor geführt. Dieser Reaktor enthielt 3 g des Dehydrierkatalysators aus Beispiel 23 (Korngrösse: 0.315 - 1 mm). Die Reaktortemperatur war 280°C. Nach 335 h Reaktionszeit enthielt die organische Phase des Produkts bei quantitativem MPDA- und MPI-Umsatz 94.6% PIC und 5.4% organische Verunreinigungen (GC-Fl.-%). Eine Katalysatordesaktivierung war nicht zu beobachten.

## Patentansprüche

1. Verfahren zur Herstellung von 3-Methylpiperidin aus 2-Methyl-1,5-diaminopentan in der Gasphase, dadurch gekennzeichnet, das gasförmiges 2-Methyl-1,5-diaminopentan bei einer Temperatur von 300 - 400 °C und einem Druck von 0 bis 10 bar Überdruck ohne Zusatz von Ammoniak über einen Katalysator geleitet wird, der aus aktiviertem Al₂O₃, einem Aluminium-Siliciummischoxid oder einem natürlichen oder synthetischen Zeolith besteht, an der Oberfläche ein Verhältnis von sauren zu basischen Zentren von über 2 aufweist und dessen spezifische Oberfläche über 40 m²/g liegt.

2. Verfahren zur Herstellung von 3-Methylpyridin, dadurch gekennzeichnet, dass zunächst gemäss Anspruch 1 aus 2-Methyl-1,5-diaminopentan 3-Methylpiperidin hergestellt und dieses anschliessend über einen zweiten Katalysator als Dehydrierungskatalysator geleitet wird.

3. Verfahren nach Anspruch 2, dadurch gekennzeichnet, dass die Dehydrierung bei 220 - 400 °C durchgeführt wird.

4. Verfahren nach Anspruch 2 oder 3, dadurch gekennzeichnet, dass als Dehydrierungskatalysator ein Edelmetall auf einem Träger verwendet wird.

5. Verfahren nach Anspruch 4, dadurch gekennzeichnet, dass als Edelmetall Palladium oder Platin eingesetzt wird.

6. Verfahren nach Anspruch 5, dadurch gekennzeichnet, dass als Dehydrierungskatalysator Palladium auf einem amorphen Silicium-Aluminiumoxid, hergestellt durch Ionenaustausch mit einem löslichen Palladiumkomplex, eingesetzt wird.

## Claims

1. A method for preparing 3-methylpiperidine from 2-methyl-1,5-diaminopentane in the vapour phase, characterised in that gaseous 2-methyl-1,5-diaminopentane, at a temperature of from 300 to 400°C and at a pressure of from 0 to 10 bar excess pressure, without addition of ammonia is passed over a catalyst which consists of activated Al₂O₃, of an aluminium-silicon mixed oxide or of a natural or synthetic zeolite, which possesses at its surface a ratio of acidic to basic centres of more than 2 and which has a specific surface of more than 40 m²/g.

2. A method for preparing 3-methylpyridine, characterised in that firstly 3-methylpiperidine is prepared from 2-methyl-1,5-diaminopentane according to claim 1 and this is then passed over a second catalyst as dehydrogenation catalyst.

3. The method according to claim 2, characterised in that the dehydrogenation is carried out at 220 to 400°C.

4. The method according to claim 2 or 3, characterised in that the dehydrogenation catalyst used is a precious metal on a support.

5. The method according to claim 4, characterised in that the precious metal used is palladium or platinum.

6. The method according to claim 5, characterised in that the dehydrogenation catalyst used is palladium on an amorphous silicon-aluminium oxide, prepared by ion exchange with a soluble palladium complex.

## Revendications

1. Procédé pour la préparation de 3-méthylpipéridine à partir de 2-méthyl-1,5-diaminopentane dans la phase gazeuse, caractérisé en ce que le 2-méthyl-1,5-diaminopentane gazeux est amené à une température de 300-400°C et à une pression de 0 à 10 bars de surpression sans addition d'ammoniac sur un catalyseur qui est constitué par de l'Aℓ₂O₃ activé, un oxyde mixte silicium-aluminium ou une zéolithe naturelle ou synthétique, présentant un rapport entre les centres acides et les centres basiques supérieur à 2 et dont la surface spécifique est supérieure à 40 m²/g.

2. Procédé pour la préparation de 3-méthylpyridine, caractérisé en ce que selon la revendication 1, on prépare d'abord la 3-méthylpipéridine à partir de 2-méthyl-1,5-diaminopentane et consécutivement, on amène celle-ci sur un deuxième catalyseur en tant que catalyseur de déshydrogénation.

3. Procédé selon la revendication 2, caractérisé en ce que la déshydrogénation est conduite à une température de 220-400°C.

4. Procédé selon la revendication 2 ou 3, caractérisé en ce qu'en tant que catalyseur de déshydrogénation, on utilise un métal noble sur un support.

5. Procédé selon la revendication 4, caractérisé en ce qu'en tant que métal noble, on met en oeuvre du palladium ou du platine.

6. Procédé selon la revendication 5, caractérisé en ce qu'en tant que catalyseur de déshydrogénation, on met en oeuvre du palladium sur un oxyde silicium-aluminium amorphe, préparé par échange ionique avec un complexe palladium soluble.
